# Europäisches Patentamt
## European Patent Office
### Office européen des brevets

(19)

(11) Veröffentlichungsnummer: **0 024 316**
**B1**

(12)

# EUROPÄISCHE PATENTSCHRIFT

(45) Veröffentlichungstag der Patentschrift:
**27.04.83**

(51) Int. Cl.³: **A 61 N 1/32,** A 61 N 1/08

(21) Anmeldenummer: **80104487.6**

(22) Anmeldetag: **29.07.80**

(54) **Elektromedizinisches Gerät zur Interferenzstrom-Behandlung.**

(30) Priorität: **03.08.79 DE 2931638**

(43) Veröffentlichungstag der Anmeldung:
**04.03.81 Patentblatt 81/9**

(45) Bekanntmachung des Hinweises auf die Patenterteilung:
**27.04.83 Patentblatt 83/17**

(84) Benannte Vertragsstaaten:
**AT BE CH FR GB IT LI NL SE**

(56) Entgegenhaltungen:

**DE-A-2 255 578**
**DE-B-2 159 437**
**FR-A-2 175 703**
**FR-A-2 340 743**
**US-A-4 148 321**

(73) Patentinhaber: **SIEMENS AKTIENGESELLSCHAFT,
Berlin und München Wittelsbacherplatz 2,
D-8000 München 2 (DE)**

(72) Erfinder: **Hudek, Karl, verstorben, (DE)**
Erfinder: **Kusserow, Bernd, Kulmbacher Strasse 12,
D-8520 Erlangen (DE)**

ACTORUM AG

## Elektromedizinisches Gerät zur Interferenzstrom-Behandlung

Die Erfindung bezieht sich auf ein elektromedizinisches Gerät zur Interferenzstrombehandlung mit wenigstens zwei Stromkreisen, welche ein gemeinsames Regelglied zur Intensitätseinstellung aufweisen, wobei die Stromkreise entweder von einem einzigen Generator zur digitalen Erzeugung von Signalfrequenzen angesteuert und über Phasenregelkreise miteinander verbunden sind oder von mehreren Generatoren zur digitalen Erzeugung von Signalfrequenzen geringer Abweichung angesteuert werden.

Bei Reizstromgeräten nach dem Interferenzprinzip soll eine Intensitätsregelung in den einzelnen Stromkreisen simultan und gleichmässig möglich sein; das heisst zwischen den Kanälen des Reizstromgerätes dürfen keine bemerkbaren Gleichlauffehler auftreten.

Bisher wurde die Stromintensität der Stromkreise von Mehrkreisinterferenzgeräten mit Mehrfachpotentiometern geregelt. Im Prinzip werden dabei von der Bedienungsperson mit einem Drehknopf gleichzeitig mehrere Potentiometer betätigt. Der Gleichlauffehler üblicher Mehrfachpotentiometer liegt aber in etwa in der Grössenordnung von $\pm 3$ dp. Effektiv können sich dadurch schon unerwünschte Verschiebungen im Intensitätsverlauf der einzelnen Kanäle ergeben. Das gleiche gilt sinngemäss dann, wenn der Intensitätsverlauf der Stromkreise durch die Steilheit von Feldeffekttransistoren gesteuert wird. Da die Steilheit und die sog. «Pinch-Off-Spannungen» solcher Bauelemente stark streuen kann, muss in diesem Fall mit erheblichem Aufwand an Justierelementen und Abgleichszeit gerechnet werden.

Der Erfindung liegt daher die Aufgabe zugrunde, ein elektromedizinisches Gerät zur Interferenzstrom-Behandlung zu schaffen, bei dem mit möglichst geringem technischem Aufwand die Intensitätsregelung der einzelnen Stromkreise vollkommen gleichmässig möglich ist.

Die Aufgabe wird bei einem Gerät der eingangs genannten Art erfindungsgemäss dadurch gelöst, dass in jedem der Stromkreise ein von den Signalfrequenzen gesteuerter Schalttransistor angeordnet ist, dessen Kollektorspannung im Schaltbetrieb entweder Nullwert oder Betriebsspannungswert aufweist, wozu alle Schalttransistoren kollektorseitig am gemeinsamen Regelglied angeschaltet sind, so dass sich die Grösse der Schaltsignale der Schalttransistoren aller Stromkreise in Abhängigkeit von der Einstellung des Regelgliedes gleichmässig ändert, wobei den Schalttransistoren Tiefpassfilter nachgeschaltet sind, welche aus den Schaltsignalen sinusförmige Signalkurven der entsprechenden Signalfrequenz erzeugen.

Da die Signalfrequenzen digital erzeugt werden, also als Rechteckspannungen vorliegen, können die Transistoren als Schalter verwendet werden, die von der Rechteckspannung der Generatoren für die verschiedenen Stromkreise durchgeschaltet werden. Dabei ist die Rechteckspannung, die kollektorseitig an den Transistoren ansteht eine Funktion der Reglerstellung, die für alle Stromkreise identisch ist. Vorzugsweise ist das Regelglied ein Potentiometer mit nachgeschaltetem Operationsverstärker in Rückkopplung, so dass durch den niederohmigen Ausgang in den parallelgeschalteten Kanälen der Einzeltransistoren kein Übersprechen erfolgen kann. Aus den digitalen Signalwerten, d.h. den Rechteckspannungen lassen sich durch den Kollektorstrecken nachgeschalteten Tiefpässen entsprechend Sinusspannungen erzeugen. Diese Sinusspannungen werden durch identische Verstärker nachverstärkt, so dass an den Ausgängen mit Elektrode die Spannungen für die erwünschten Interferenzströme im Patienten zur Verfügung stehen.

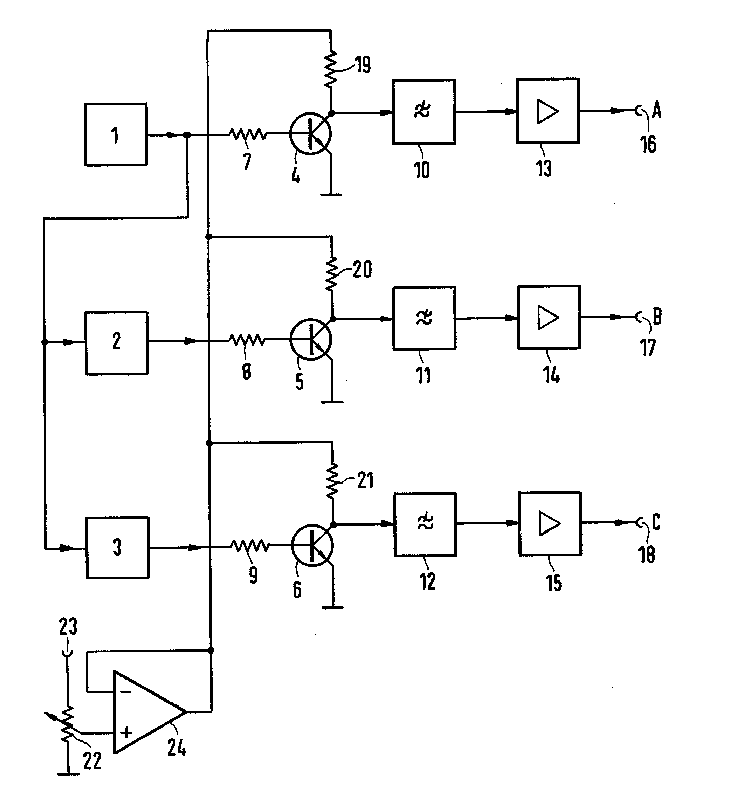

Weitere Einzelheiten und Vorteile der Erfindung ergeben sich aus nachfolgender Figurenbeschreibung eines Ausführungsbeispiels anhand der Zeichnung in Verbindung mit weiteren Unteransprüchen.

Die Figur zeigt schaltbildmässig ein Interferenzstromgerät mit simultaner, identischer Intensitätsregelung der Stromkreise.

In der Figur bedeuten A der Kanal eines ersten Stromkreises, B der Kanal eines zweiten Stromkreises und C der Kanal eines dritten Stromkreises, welche über Elektroden an entsprechenden Stellen des Patientenkörpers zur Ausbildung von Interferenzströmen im Patienteninneren applizierbar sind. Mit 1 ist ein Signalgenerator zur Erzeugung von Signalfrequenzen etwa im Mittelfrequenzbereich, z.B. zwischen 1000 und 100 000 Hz gekennzeichnet. Der Signalgenerator 1 steuert den Stromkreis A direkt an. Parallel geschaltet sind dem Generator 1 Phasenregelkreise 2 und 3 (sog. phase-locked-loops), mit denen rhythmische Phasenverschiebungen in den Stromkreisen B und C eingestellt werden können. Alternativ dazu können separate Generatoren für jeden Stromkreis verwendet werden, die die zur Interferenz geeigneten Frequenzen geringer Unterschiede erzeugen. In den drei Stromkreisen ist je ein Transistor 4, 5 und 6 angeordnet, welche im Schaltbetrieb arbeiten. Speziell sind npn-Transistoren in Emitterschaltung dargestellt, wobei die Basis der Transistoren 4 bis 6 mit Widerständen 7 bis 9 beschaltet ist. Die Emitterstrecke der Transistoren 4 bis 6 liegt jeweils auf Massepotential. Den Transistoren 4 bis 6 sind kollektorseitig jeweils Tiefpässe 10 bis 12 und identische Verstärker 13 bis 15 nachgeschaltet, von denen Stromausgänge 16 bis 18 der Stromkreise A bis C angesteuert werden.

Die Kollektorausgänge der Transistoren 4, 5 und 6 sind jeweils über Widerstände 19 bis 21 an eine gemeinsame Steuerleitung angeschlossen. Diese Steuerleitung ist über ein Potentiometer 22 als Intensitätsregler an eine Spannungsquelle 23 angeschaltet. Dem Potentiometer 22 ist ein rückgekoppelter Operationsverstärker 24 als Impedanzwandler angeschlossen, so dass der Ausgang an die gemeinsame kollektorseitige Steuerleitung der Transistoren 4 bis 6 niederohmig ist. Dadurch wird eine gegenseitige Beeinflussung der Kanäle der einzelnen Stromkreise verhindert.

Die so beschriebene Schaltung hat folgende Funktion:

Vom Generator 1 liegen direkt bzw. über die Phasenregelkreise 2 und 3 Rechtecksignale mit sich rhythmisch verändernder Phasenlage an den Signalleitungen für die einzelnen Kanäle A bis C an. Die Transistoren 4 bis 6 sind jeweils bis zur Sättigung ausgesteuert, so dass sie in Abhängigkeit der digitalen vorliegenden Signale im Schaltbetrieb mit vorgegebener Frequenz arbeiten. Die Kollektorspannungen der Transistoren 4 bis 6 sind dann entweder Null oder Betriebsspannung. Die Betriebsspannung der Transistoren 4 bis 6 wird durch das einzige Potentiometer 22 gleichlaufend für alle Transistoren auf den gewünschten Wert eingestellt. Durch den rückgekoppelten Operationsverstärker 24 ist ein Übersprechen der Einzelkanäle mit den Transistoren 4 bis 6 verhindert, so dass kollektorseitig tatsächlich jeweils die gleiche Rechteckspannung als Funktion der Reglerstellung ansteht. Diese Rechteckspannungen werden über die Tiefpässe 10 bis 12 in Sinusspannungen umgeformt und in den Verstärkern 13 bis 15 verstärkt. Die Ausgänge 16 bis 18 der Kanäle A, B, C können über Elektroden unmittelbar am Patientenkörper appliziert werden, in dem sich die erwünschten Interferenzströme ausbilden.

In einem anderen Ausführungsbeispiel sind die Transistoren einzelner Stromkreise kollektorseitig zusätzlich mit einem Spannungsteiler beschaltet. Dadurch lässt sich die Intensität einzelner Stromkreise ändern, wobei die gleichlaufende relative Änderung in Abhängigkeit von der Reglerstellung vorhanden bleibt. Zweckmässigerweise ist dabei dem Spannungsteiler ein Impedanzwandler nachgeschaltet. Es ist auch möglich durch Schaltung eines invertierenden Verstärkers eine solche Regelung zu schaffen, bei der sich die Intensitäten in den einzelnen Stromkreisen definiert gegenläufig verändern.

### Patentansprüche

1. Elektromedizinisches Gerät zur Unterferenzstrombehandlung mit wenigstens zwei Stromkreisen (A, B, C), welche ein gemeinsames Regelglied (22, 24) zur Intensitätseinstellung aufweisen, wobei die Stromkreise entweder von einem einzigen Generator (1) zur digitalen Erzeugung von Signalfrequenzen angesteuert und über Phasenregelkreise (2, 3) miteinander verbunden sind oder von mehreren Generatoren zur digitalen Erzeugung von Signalfrequenzen geringer Abweichung angesteuert werden, dadurch gekennzeichnet, dass in jedem der Stromkreise (A, B, C) ein von den Signalfrequenzen gesteuerter Schalttransistor (4, 5, 6) angeordnet ist, dessen Kollektorspannung im Schaltbetrieb entweder Nullwert oder Betriebsspannungswert (23) aufweist, wozu alle Schalttransistoren (4, 5, 6) kollektorseitig am gemeinsamen Regelglied (22, 24) angeschaltet sind, so dass sich die Grösse der Schaltsignale der Schalttransistoren (4, 5, 6) aller Stromkreise (A, B, C) in Abhängigkeit von der Einstellung des Regelgliedes (22, 24) gleichmässig ändert, wobei den Schalttransistoren (4, 5, 6) Tiefpassfilter (10, 11, 12) nachgeschaltet sind, welche aus den Schaltsignalen sinusförmige Signalkurven der entsprechenden Signalfrequenz erzeugen.

2. Elektromedizinisches Gerät nach Anspruch 1, dadurch gekennzeichnet, dass das Regelglied ein Potentiometer (22) mit nachgeschaltetem Operationsverstärkrer (24) ist, der durch Rückkopplung einen niederohmigen Ausgang hat.

3. Elektromedizinisches Gerät nach Anspruch 2, dadurch gekennzeichnet, dass den Tiefpassfiltern (10, 11, 12) in den einzelnen Stromkreisen (A, B, C) jeweils identische Verstärker (13, 14, 15) nachgeschaltet sind.

4. Elektromedizinisches Gerät nach Anspruch 1, dadurch gekennzeichnet, dass an die Transistoren (4, 5, 6) in den einzelnen Stromkreisen (A, B, C) kollektorseitig Spannungsteiler zur Teilung der Steuerspannung anschaltbar sind, so dass sich die Grösse der Schaltsignale der einzelnen Transistoren (4, 5, 6) in Abhängigkeit von der Reglerstellung in definiertem Verhältnis ändert.

5. Elektromedizinisches Gerät nach Anspruch 4, dadurch gekennzeichnet, dass wenigstens einem Spannungsteiler ein Impedanzwandler nachgeschaltet ist.

6. Elektromedizinisches Gerät nach Anspruch 4, dadurch gekennzeichnet, dass wenigstens einem Spannungsteiler ein invertierender Verstärker nachgeschaltet ist.

### Claims

1. Electro-medical apparatus for interference current treatment comprising at least two circuits (A, B, C) which possess a common regulating element (22, 24) for intensity adjustment, where the circuits are either driven by one single generator (1) serving to generate digital signal frequencies connected via phase regulating circuits (2, 3), or are driven by a plurality of generators which serve to generate digital signal frequencies of low deviation, characterised in that in each of the circuits (A, B, C) there is arranged a switching transistor (4, 5, 6) controlled by the signal frequencies and whose collector voltage during the switching operation is either zero or the operating voltage value (23), for which purpose all the switching transistors (4, 5, 6) are connected at the collector end to the common regulating circuit (22, 24) so that the value of the switching signals of the switching transistors (4, 5, 6) of all the circuits (A, B, C) changes uniformly in dependence upon the setting of the regulating element (22, 24), the switching transistors (4, 5, 6) being followed by low-pass filters (10, 11, 12) which produce from the switching signals sinusoidal signal waveforms of the corresponding signal frequency.

2. Electro-medical apparatus as claimed in Claim 1, characterised in that the regulating element consists of a potentiometer (22) followed by an operational amplifier (24) provided with feedback to exhibit a low-ohmic output.

3. Electro-medical apparatus as claimed in Claim 2, characterised in that the low-pass filters (10, 11, 12) in the individual circuits (A, B, C) are each followed by identical amplifiers (13, 14, 15).

4. Electro-medical apparatus as claimed in Claim 1, characterised in that the transistors (4, 5, 6) in the individual circuits (A, B, C) can be connected at the collector end to voltage dividers which serve to divide the control voltage, so that the value of the switching signals of the individual transistors (4, 5, 6) changes in a determinate ratio in dependence upon the setting of the regulator.

5. Electro-medical apparatus as claimed in Claim 4, characterised in that at least one voltage divider is followed by an impedance transformer.

6. Electro-medical apparatus as claimed in Claim 4, characterised in that at least one voltage divider is followed by an inverting amplifier.

## Revendications

1. Appareil électromédical pour le traitement par courants d'interférence, qui comporte au moins deux circuits de courant (A, B, C) qui comprennent un organe de réglage commun (22, 24) pour le réglage de l'intensité, les circuits de courant étant soit reliés entre eux par l'intermédiaire de régulateurs de phases (2, 3) et commandés par un générateur unique (1) pour produire numériquement des fréquences de signaux, soit commandées par plusieurs générateurs pour produire numériquement des fréquences de signaux différant faiblement, caractérisé par le fait que chaque circuit de courant (A, B, C) comporte un transistor de commutation (4, 5, 6), commandé par les fréquences des signaux, dont la tension de collecteur dans le mode de commutation possède soit une valeur nulle soit la valeur de la tension de service (23), les collecteurs de tous les transistors de commutation (4, 5, 6) étant à cet effet reliés à l'organe de réglage commun (22, 24), de manière que la valeur des signaux de commutation des transistors de commutation (4, 5, 6) de tous les circuits de courant (A, B, C) varie uniformément en fonction du réglage de l'organe de réglage (22, 24), des filtres passe-bas (10, 11, 12), qui produisent des courbes de signaux sinusoïdales possédant la fréquence de signaux correspondante, étant montés en aval des transistors de commutation (4, 5, 6).

2. Appareil électromédical suivant la revendication 1, caractérisé par le fait que l'organe de réglage est constitué par un potentiomètre (22) en aval duquel est monté un amplificateur opérationnel (24), qui possède une sortie de faible valeur ohmique, grâce à une contre-réaction.

3. Appareil électromédical suivant la revendication 2, caractérisé par le fait que dans les circuits de courant individuels (A, B, C), en aval des filtres passe-bas (10, 11, 12), sont respectivement montés des amplificateurs identiques (13, 14, 15).

4. Appareil électromédical suivant la revendication 1, caractérisé par le fait que dans les circuits de courant individuels (A, B, C), au collecteur des transistors (4, 5, 6) peuvent être reliés des diviseurs de tensions destinés à diviser la tension de commande, de manière que la valeur des signaux de commutation des transistors individuels (4, 5, 6) varie dans un rapport défini en fonction de la position de l'organe de réglage.

5. Appareil électromédical suivant la revendication 4, caractérisé par le fait qu'un convertisseur d'impédance est monté en aval d'au moins un diviseur de tension.

6. Appareil électromédical suivant la revendication 4, caractérisé par le fait qu'un amplificateur inverseur est monté en aval d'au moins un diviseur de tension.